(19) Europäisches Patentamt
European Patent Office
Office européen des brevets

(11) Veröffentlichungsnummer: **0 083 439**
**B1**

(12) # EUROPÄISCHE PATENTSCHRIFT

(45) Veröffentlichungstag der Patentschrift: **26.07.89**

(51) Int. Cl.⁴: **A 61 K 31/675**, A 61 K 31/10, A 61 K 31/19, C 07 F 9/65 // (A61K31/675, 31:19, 31:10)

(21) Anmeldenummer: **82111907.0**

(22) Anmeldetag: **22.12.82**

(54) **4-Sulfido-oxazaphosphorine und deren Verwendung bei der Krebsbekämpfung und zur Immunsuppression.**

(30) Priorität: 31.12.81 DE 3151977
11.06.82 DE 3222006

(43) Veröffentlichungstag der Anmeldung:
13.07.83 Patentblatt 83/28

(45) Bekanntmachung des Hinweises auf die Patenterteilung:
26.07.89 Patentblatt 89/30

(84) Benannte Vertragsstaaten:
BE CH DE FR GB IT LI NL SE

(56) Entgegenhaltungen:
GB-A- 2 095 256

Makromol. Chem. 180, (1979), pp. 1125-1131
CANCER, CHEMOTHERAPY AND PHARMACOLOGY, Band 3, 1979, Springer Verlag, P. PETER et al.: "Synthesis and Preliminary Antitumor Evaluation of 4-(SR)-Sulfido-Cyclophosphamides", pp. 181-188
CHEMICAL ABSTRACTS, Band 93, Heft 21, 24. November 1980, p. 52, Zusammenfassung Nr. 197752w, Columbus, Ohio, US, L.LEVY et al.: "The influence of N.acetylsysteine (NAC) on cyclo-phosphamide (CPA) action on transplanted lymphomas"
CANCER TREATMENT REPORT, Band 60, Heft 4, April 1976, U. DRAEGER et al.: "Deactivation of Cyclophosphamide (NSC-26271) Metabolites by

(73) Patentinhaber: ASTA Pharma AG, Weismüllerstrasse 45, D-6000 Frankfurt am Main 1 (DE)

(72) Erfinder: Hohorst, Hans-Jürgen, Prof.Dr.Dr., Birkenweg 18, D-6550 Marburg-Marbach (DE)
Erfinder: Gernot, Peter, Dr., Dr.-Carl-Henss-Strasse 28, D-6369 Nidderau 2 (DE)
Erfinder: Voelcker, Georg, Dr., Johann-Peter-Bach-Strasse 1, D-6369 Nidderau-Windecken (DE)
Erfinder: Wrabetz, Erhardt, Praunheimer Weg 34, D-6000 Frankfurt am Main (DE)

(56) Entgegenhaltungen: (Fortsetzung)
Sulfhydryl Compounds 1,2", pp. 355-359
TETRAHEDRON LETTERS, no. 10, März 1979, Pergamon Press, pp. 883-886, GB, T. HIRANO et al.: "Synthesis of activated cyclophosphamide derivatives bearing functional groups"

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Verbindungen der allgemeinen Formel

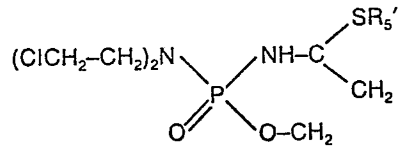

worin $R_5'$ die Gruppe $-(CH_2)_n-OH$ bedeutet und n 2, 3, 4 oder 6 ist sind bekannt. Diese Verbindungen besitzen eine Antitumor-Wirkung, sind jedoch zugleich sehr toxisch (siehe Tetrahedron Letters Nr. 10 (1979) Seiten 883–886; Cancer Chemother. Pharmacol. 3 (1979) Seiten 181–188).

Weiterhin wird in Cancer Treatment Reports 60 (1976) Seiten 355–359 die Entstehung von Verbindungen der Formel

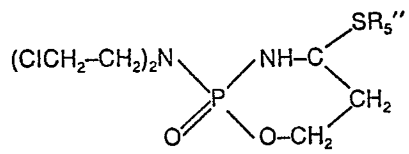

worin $R_5''$ die Gruppe $-CH_2-CH(NH_2)-CO_2H$ oder $-CH_2-CH(NHCOCH_3)-CO_2H$ ist aus 4-Hydroxy-cyclophosphamid und Cystein N-Acetyl-cystein und Glutathion beschrieben.

Die Erfindung betrifft die Verwendung von 4-Sulfidooxazaphosphorinen der allgemeinen Formel

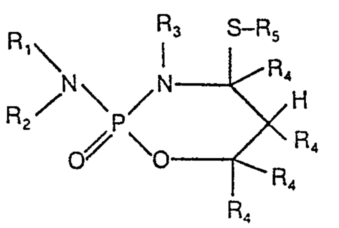

I

worin

$R_1$, $R_2$ und $R_3$, die gleich oder verschieden voneinander sein können, Wasserstoff, Methyl, Ethyl, 2-Chlorethyl oder 2-Methansulfonyloxyethyl darstellen und dabei mindestens zwei dieser Reste 2-Chlorethyl und/oder 2-Methansulfonyloxyethyl sind,

$R_4$ Wasserstoff oder Methyl ist und

$R_5$ ein $C_2$–$C_6$-Hydroxy-alkylrest oder ein $C_2$–$C_6$-Mercaptoalkylrest ist, wobei jeder dieser Reste auch eine zusätzliche Mercaptogruppe enthalten kann oder worin $R_5$ eine Carboxy-$C_1$–$C_{10}$-alkylgruppe, eine Carb-$C_1$–$C_6$-alkoxy-$C_1$–$C_{10}$-alkylgruppe, eine 2-Amino-2-carboxy-ethylgruppe, eine 2-Amino-2-carb-$C_1$–$C_6$-alkoxy-ethylgruppe, eine 2-$C_2$–$C_6$-Alkanoylamino-2-carboxyethylgruppe, eine 2-$C_2$–$C_6$-Alkanoylamino-2-carb-$C_1$–$C_6$-alkoxy-ethylgruppe, den Glutathionylrest, eine $C_2$–$C_6$-Sulfoalkylgruppe oder eine $C_2$–$C_6$-Sulfoalkylgruppe, welche eine Mercaptogruppe enthält, bedeutet sowie deren physiologisch verträglichen Salzen in Kombination mit Thioverbindungen der allgemeinen Formel

$$R_6SR_7 \qquad II$$

worin $R_6$ der Glutathionylrest ist oder einen geraden oder verzweigten $C_2$–$C_6$-Alkylrest darstellt, welcher ein- oder zweifach durch Hydroxygruppen, Mercaptogruppen, Aminogruppen, $C_2$–$C_6$-Alkanoylaminogruppen, Sulfogruppen, Carb-$C_1$–$C_6$-alkoxygruppen oder Carboxygruppen substituiert ist und $R_7$ Wasserstoff bedeutet oder worin $R_7$ auch eine $C_2$–$C_6$-Sulfoalkylthiogruppe sein kann, falls $R_6$ eine $C_2$–$C_6$-Sulfoalkylgruppe darstellt beziehungsweise den physiologisch verträglichen Salzen solcher Thioverbindungen der Formel II zur Herstellung eines Arzneimittels für die therapeutische Anwendung zur Bekämpfung von Krebs und zur Immunsuppression.

Durch die erfindungsgemäße Verwendung von Verbindungen der Formel I und Verbindungen der Formel II wird eine gute cancerotoxische Wirkung unter gleichzeitiger erheblicher Verminderung der allgemeinen Toxizität erreicht, so daß durch die erfindungsgemäße Kombination eine günstige lokoregionale Chemotherapie und eine optimierte systemische Chemotherapie von Krebskranken ermöglicht wird.

Die Verbindungen der Formel I können in verschiedenen stereoisomeren Formen vorliegen, zum Beispiel als Racemate, als optisch aktive Verbindungen oder als Diastereomere (cis- und trans-Formen). Unter Verbindungen der Formel I werden sämtliche mögliche stereoisomere Verbindungen und deren Mischungen verstanden. Die Herstellung solcher Verbindungen der Formel I kann beispielsweise nach der Methode erfolgen, die in der deutschen Patentanmeldung P 3 220 432.9 beziehungsweise der deutschen Patentanmeldung P 3 111 428.8 angegeben ist.

Beispielsweise kann die Herstellung von Verbindungen der Formel I aus den bekannten 4-Hydroxy- oder 4-Alkoxyoxazaphosphorinen der Formel III

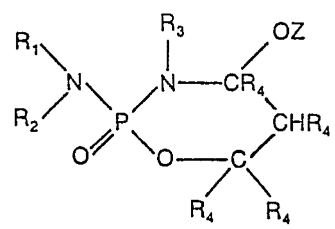

worin die Reste $R_1$, $R_2$, $R_3$ und $R_4$ die angegebenen Bedeutungen haben und Z Wasserstoff oder eine $C_1$–$C_4$-Alkylgruppe ist mit den entsprechenden Thiolen der Formel $HSR_5$ erfolgen. $R_5$ hat hierbei die angegebene Bedeutung. Die Umsetzung erfolgt in einem Lösungsmittel, zweckmäßig in Gegenwart saurer Katalysatoren. Als saure Katalysatoren kommen beispielsweise in Betracht: Trichloressigsäure, Trifluoressigsäure oder ganz allgemein anorganische oder organische Säuren oder auch Lewissäuren wie $AlCl_3$, $ZnCl_2$, $TiCl_4$ (siehe Tetrahedron Letters Nr. 10 (1979) Seiten 883–886 oder auch Cancer Chemother. Pharmacol. 3 (1979) Seiten 181–188).

Als Lösungsmittel kommen beispielsweise in Frage: Wasser, Methylenchlorid, Alkohole, insbesondere Niederalkanole wie Methanol, Ethanol, Propanol oder Isobutanol, niedere Alkylketone wie insbesondere Aceton, Dimethylformamid (DMF) oder ähnliche Lösungsmittel beziehungsweise Gemische aus mehreren solcher Lösungsmittel. Die Reaktion wird beispielsweise bei Temperaturen im Bereich von −30 °C und +40 °C durchgeführt, das heißt gegebenenfalls unter Kühlen, bei Raumtemperatur oder unter Erwärmen.

Falls der Rest $R_5$ der Verbindungen der Formel I eine $C_2$–$C_6$-Sulfoalkylgruppe bedeutet, können die entsprechenden sulfonsauren Salze durch Neutralisieren der Sulfonsäuregruppen mit der entsprechenden Base erhalten werden. Weiterhin kann in einem solchen Salz das Kation an einem Ionenaustauscher in bekannter Weise gegen ein anderes Kation ausgetauscht werden. Es kann natürlich auch die Verbindung III direkt mit dem entsprechenden sulfonsauren Salz eines $C_2$–$C_6$-Sulfoalkylmercaptans beziehungsweise dem Salz eines $C_2$–$C_6$-Sulfoalkylmercaptans, welches eine weitere Mercaptogruppe enthält, wie oben angegeben umgesetzt werden.

Die als Ausgangsmaterial verwendeten Verbindungen der Formel III sind bekannt und können kristallin oder als Rohprodukt eingesetzt werden und lassen sich wie folgt in bekannter Weise synthetisieren:

4-Hydroxy-oxazaphosphorine werden durch Reduktion der 4-Hydroperoxy-Derivate erhalten (zum Beispiel A. Takamizawa u.a., J. Amer. Chem. Soc. 95, 589 (1973)). 4-Alkoxy-oxazaphosphorine bilden sich unter saurer Katalyse aus den Hydroxyderivaten in dem entsprechenden Alkohol. Die Sulfoalkylthiole können beispielsweise durch Umsetzung des entsprechenden Natriumbromalkansulfonats mit Thioharnstoff zum Thiuroniumsalz, dessen Spaltung mit Ammoniak erhalten werden. Die so erhaltene Mercaptoalkansulfonsäure kann dann in das gewünschte Sulfonsäure-Salz übergeführt werden. Von den so erhaltenen Oxazaphosphorin-Derivaten lassen sich beispielsweise die racemischen cis- und trans-Isomere herstellen.

Der Hydroxyalkylrest, der Mercaptoalkylrest, der Carboxy-$C_1$–$C_{10}$-alkylrest sowie der Sulfoalkylrest (Gruppe -alkyl-$SO_3H$) bei den Verbindungen der Formel I und Formel II kann gerade oder verzweigt sein. Ebenfalls kann die $C_1$–$C_6$-Alkoxygruppe als Bestandteil der Carb-$C_1$–$C_6$-alkoxy-gruppe (Carbalkoxygruppe worin die Alkoxygruppe aus 1 bis 6 C-Atomen besteht) gerade oder verzweigt sein. Insbesondere besteht eine solche $C_1$–$C_6$-Alkoxygruppe aus 1 bis 4, vorzugsweise 1 bis 2 C-Atomen.

Die Hydroxygruppe, die Mercaptogruppe, die Carboxygruppe, die Carb-$C_1$–$C_6$-alkoxygruppe sowie die Sulfonsäuregruppe befinden sich vorzugsweise in W-Stellung des jeweiligen Alkylrestes. Insbesondere handelt es sich um Alkylreste mit 2 bis 4, vorzugsweise 2 C-Atomen. Desgleichen handelt es sich bei dem $C_2$–$C_6$-Alkylrest der Verbindungen der Formel II vorzugsweise um einen solchen mit 2 bis 4 C-Atomen. Unter Glutathionylrest wird der einwertige Rest verstanden, der aus Glutathion nach Entfernen der –SH–Gruppe verbleibt.

Falls $R_5$ eine 2-$C_2$–$C_6$-Alkanoylamino-2-carboxyethylgruppe ist, bedeutet Alkanoyl beispielsweise Acetyl, Propionyl, Butyryl, Isobutyryl, tert.-Butyryl. Dasselbe gilt hinsichtlich $C_2$–$C_6$-Alkanoyl, falls der Rest $R_6$ der Formel II einen $C_2$–$C_6$-Alkylrest darstellt, welcher durch eine $C_2$–$C_6$-Alkanoylaminogruppe substituiert ist.

Falls $R_5$ eine Carboxy-$C_1$–$C_{10}$-alkylgruppe oder eine Carb-$C_1$–$C_6$-alkoxy-$C_1$–$C_{10}$-alkylgruppe bedeutet, besteht die $C_1$–$C_{10}$-alkylgruppe vorzugsweise aus 2 bis 6 C-Atomen, insbesondere 2, 3, 4, 5 oder 6 C-Atomen. Die $C_1$–$C_6$-Alkoxygruppe als Bestandteil der Carb-$C_1$–$C_6$-alkoxygruppe besteht ganz allgemein vorzugsweise aus 1 bis 4 C-Atomen, vorzugsweise einem oder 2 C-Atomen.

Falls in der Formel II der Rest $R_7$ eine $C_2$–$C_6$-Sulfoalkylthiogruppe ist, handelt es sich um Dithio-di-$C_2$–$C_6$-alkansulfonsäuren der Formel HO-$SO_2$–alk–S–S–alk–$SO_2$–OH, wobei alk ein geradkettiger oder verzweigter Alkylenrest mit 2–6 C-Atomen ist; vorzugsweise ist alk die Gruppe –$CH_2$–$CH_2$.

Die an der $C_2$–$C_6$-Sulfoalkylgruppe (Rest $R_5$ von Formel I) befindliche Mercaptogruppe sitzt beispielsweise an dem 1-, 2-, 3-, 4- oder 5-ständigen Kohlenstoffatom der Alkylenkette (Zählung beginnt von der Sulfonsäuregruppe aus). Die Mercaptogruppe kann sich jedoch nicht an dem Kohlenstoffatom der Alkylenkette befinden, das die Bindung mit dem Oxazaphosphorinyl-(4)-thio-rest herstellt.

Insbesondere handelt es sich um solche Verbindungen der Formel I, worin $R_5$ die angegebenen Bedeutungen hat und die Reste $R_1$–$R_4$ die folgenden Bedeutungen haben:

$R_1$ und $R_2$ = 2-Chlor-ethyl, $R_3$ und $R_4$ = H;
$R_1$, $R_2$ und $R_3$ = 2-Chlor-ethyl, $R_4$ = H;
$R_1$ und $R_3$ = 2-Chlor-ethyl, $R_2$ und $R_4$ = H;
$R_1$ = 2-Methylsulfonyloxy-ethyl, $R_3$ = 2-Chlorethyl, $R_2$ und $R_4$ = H.

Als Thioverbindungen der Formel II kommen insbesondere solche Verbindungen der Formel II in Frage, bei denen der Rest $R_7$ Wasserstoff ist.

Falls die Verbindungen der Formel I eine Sulfonsäuregruppe enthalten, können auch therapeutisch verwendbare Salze der Verbindungen der Formel I verwendet werden, die durch Salzbildung mit dieser Sulfonsäuregruppe entstehen. Insbesondere kommen in diesen Fällen die Alkali- (Kalium, Natrium) Erdalkali- (Calcium, Magnesium) oder Ammoniumsalze in Betracht. Im Falle der Ammoniumsalze leiten sich diese insbesondere vom Ammoniak dessen 1, 2 oder 3 H-Atome auch durch Methyl-, Ethyl- oder 2-Hydroxyethylreste substituiert sein können beziehungsweise von folgenden Aminen ab: Guanidin, Morpholin, Cyclohexylamin, Ethylendiamin, Piperazin. Im Falle eines zweibasischen Kations oder eines Amins mit zwei einbasischen Kationen (zum Beispiel Ethylendiamin) handelt es sich um Salze, die 2 Moleküle der der Formel I entsprechenden sulfonsauren Verbindung enthalten. Die sulfonsauren Salze werden beispielsweise aus den entsprechenden Sulfonsäuren der Formel I und den entsprechenden Alkali- oder Erdalkalihydroxiden oder Ammoniak beziehungsweise den entsprechenden Aminen in der hierfür üblichen Weise erhalten.

Falls der Rest $R_6$ der Thioverbindungen der Formel II ein zweifach substituierter $C_2$–$C_6$-Alkylrest ist, kann dieser zwei gleiche oder zwei verschiedene der angegebenen Substituenten enthalten. Beispielsweise kommen folgende Thioverbindungen in Betracht: 2,3-Dimercaptopropanol, Penicillamin, Cystein, Cystein, dessen Carboxygruppe mit $C_1$–$C_6$-Alkanolen verestert ist, insbesondere $C_1$–$C_4$-Alkanolen wie Cysteinmethylester oder Cysteinethylester, Homocystein, 3-Mercapto-1,2-propandiol, 2,3-Dimercapto-propansulfonsäure, 1,2,3-Trimercaptopropan, 2-Mercapto-ethansulfonsäure (insbesondere als Natriumsalz), 2,2'-Dithiodi-ethansulfonsäure (insbesondere als Natriumsalz).

Als Salze der Thioverbindungen der Formel II kommen insbesondere die Salze in Frage, die von den Thioverbindungen der Formel II mit sauren Gruppen (Carboxygruppen, Sulfoalkylgruppen) gebildet werden. Es handelt sich hierbei insbesondere um die entsprechenden Alkalisalze (Na, K) oder um Ammoniumsalze. Die Ammoniumsalze leiten sich beispielsweise vom Ammoniak oder von folgenden Aminen ab: $C_1$–$C_4$-Mono-, Di- oder Trialkylaminen, die gegebenenfalls auch eine Hydroxygruppe enthalten können (zum Beispiel Mono-, Di- oder Triethanolamine), Ethylendiamin, Cholin, Betain, Colamin. Falls die Dithio-di-$C_2$–$C_6$-alkansulfonsäuren als Salze eingesetzt werden, kommen hierfür insbesondere die neutralen Alkali- beziehungsweise Ammoniumsalze in Betracht.

Die 4-Sulfido-oxazaphosphorine der Formel I können zusammen mit den Thioverbindungen der Formel II, zum Beispiel in Form einer Mischung, einer Lösung oder auch getrennt in einer Kapsel, das heißt zusammen peroral oder parenteral zur Anwendung kommen. Die Verbindungen der Formel I und II können jedoch auch getrennt, das heißt jede für sich in Form einer eigenen pharmazeutischen Zubereitung angewendet werden. Im letzteren Falle ist eine gleichzeitige Applikation oder eine Applikation zu verschiedenen Zeiten möglich. Liegen die Verbindungen der Formel I und II in verschiedenen galenischen Zubereitungen vor, werden beide Zubereitungen für eine gleichzeitige Applikation entweder peroral oder parenteral (zum Beispiel intravenös oder intraperitoneal) appliziert. Bei nicht gleichzeitiger Applikation empfiehlt es sich, die Thioverbindung der Formel II zuerst peroral oder parenteral und die Verbindung I später parenteral zu applizieren. Die Verabreichung der Thioverbindung II erfolgt dabei zweckmäßig innerhalb eines Zeitraumes von etwa 120 Minuten vor Verabreichung der Verbindung I bis etwa 120 Minuten nach der Verabreichung der Verbindung I, vorzugsweise 60 Minuten vor bis 30 Minuten nach, insbesondere 30 Minuten vor bis 10 Minuten nach Verabreichung der Verbindung I und zwar in einer Menge von mindestens 1 Mol pro 1 Mol der zu verabreichenden beziehungsweise verabreichten Menge des 4-Sulfido-oxazaphosphorins I bis höchstens der höchstverträglichen Dosis der Thioverbindungen II, im allgemeinen bis zu 10 Mol pro 1 Mol 4-Sulfido-oxazaphosphorin I.

Falls die Thioverbindung II nicht gleichzeitig mit dem 4-Sulfido-oxazaphosphorin der Formel I appliziert wird, können beispielsweise folgende Mengen an Thioverbindung parenteral oder oral gegeben werden:

5 g Cystein (42 Millimol) als Thiol II 60 Minuten vor Applikation von beispielsweise 1,4 g 4-(2-Hydroxy-ethylmercapto)-cyclophosphamid ($P_1$) oder 2,3 g 3-[2-(Bis-(2-chlorethyl)-amino-2-oxo-tetrahydro-2H-1,3,2-oxazaphosphorin-4-yl-thio]-2-mercaptopropansulfonsäure-Cyclohexylaminsalz (im folgenden wird diese Verbindung als «Cyclohexylaminsalz» bezeichnet als Verbindung I. Die Menge von 1,4 g $P_1$ beziehungsweise 2,3 g Cyclohexylaminsalz entsprechen jeweils 4,2 Millimol dieser beiden Verbindungen.

2,5 g Cystein (21 Millimol) gleichzeitig mit Applikation von 1,4 g der Verbindung $P_1$ oder 2,3 g Cyclohexylaminsalz.

5 g Cystein 30 Minuten nach Applikation von 1,4 g der Verbindung $P_1$ oder 2,3 g Cyclohexylaminsalz.

Bei der zuvor erwähnten Applikation der Thioverbindung vor und nach Applikation der 4-Sulfido-oxazaphosphorin-Verbindung ist das Molverhältnis von Thioverbindung II zu 4-Sulfido-oxazaphosphorin beispielsweise 10:1, bei der gleichzeitigen Applikation 5:1. Falls anstelle des Cysteins beispielsweise eine andere Thioverbindung II verwendet wird, errechnet sich die entsprechende Menge zum Beispiel aufgrund des zuvor angegebenen Molverhältnisses. Ebenfalls können anstelle von $P_1$ auch zum Beispiel 4,2 Millimol eines anderen 4-Sulfido-oxazaphosphorins der Formel I beziehungsweise eines entsprechenden Salzes hiervon verwendet werden.

Bei intravenöser Applikation der 4-Sulfido-oxazaphosphorine I sollte die Injektionsdauer länger als 1 Minute betragen, zum Beispiel 2 bis 10 Minuten. Das jeweils applizierte Injektionsvolumen sollte bei parenteraler Applikation, (zum Beispiel intravenös, intraperitoneal) pro Injektion etwa 0,5 bis 100 ml betragen, das heißt es sind möglichst niedriger konzentrierte Lösungen der 4-Sulfido-oxazaphosphorine zu verwenden, zum Beispiel 0,1 bis 7%ige Lösungen, vorzugsweise 0,5 bis 2%ige Lösungen der 4-Sulfido-oxazaphosphorine I. Vorzugsweise werden die Thioverbindungen der Formel II sowie die 4-Sulfido-oxazaphosphorine parenteral verabreicht. Dies gilt sowohl für getrennte als auch für gemeinsame Applikation der beiden Komponenten.

Die erfindungsgemäße Kombination von Verbindungen der Formel I mit den Thioverbindungen der Formel II zeigt beispielsweise bei intraperitonealer Injektion bei der NMRI-Maus* am 3. Tage nach Transplantation von S180 Krebszellen ($10^6$ Tumorzellen/Maus) eine gute cytostatische Wirkung (Methode nach Doldin, Johnson, Venditti, Preclinical, Characterization of Candidate Antitumor Drugs, Cancer Chemotherapy Reports, Part 2, Vol. 5, 1975).

Beispielsweise wird bei oben genannter Versuchsmethode bei einer einmaligen Dosis von 32 mg/kg 4-(2-Hydroxy-ethylmercapto)-cyclophosphamid (= $P_1$) und 58 mg/kg Cystein eine Heilung von 50% der tumortragenden Tiere erhalten; bei einer Dosis von 220 mg/kg $P_1$ und 392 mg/kg Cystein werden 95% der Tiere geheilt. Eine einmalige Dosis von 52 mg/kg Cyclohexylaminsalz und 47 mg/kg Cystein bewirkt bei derselben Versuchsmethode ebenfalls eine Heilung der Mehrzahl der tumortragenden Tiere.

Diese cytostatische Wirkung ist mit derjenigen des bekannten Arzneimittels Cyclophosphamid vergleichbar.

Die niedrigste, bereits cancerotoxisch wirksame Dosis in dem oben angegebenen Tierversuch ist beispielsweise im Falle des 4-(2-Hydroxy-ethylmercapto)-cyclophosphamids ($P_1$) und Cystein 5 mg/kg $P_1$ und 9 mg/kg Cystein intraperitoneal, im Falle der Kombination Cyclohexylaminsalz/Cystein: 8,2 mg/kg Cyclohexylaminsalz und 7,4 mg/kg Cystein intraperitoneal.

Als allgemeiner Dosisbereich für die Wirkung in obigem Tierversuch kommt beispielsweise eine Dosierung zwischen 10 mg/kg Verbindung I und 9–18 mg/kg Thioverbindung II bis 400 mg/kg Verbindung I und 360–720 mg/kg Thioverbindung II in Frage, insbesondere Dosierungen zwischen 50 mg/kg Verbindung I und 45–85 mg/kg Thioverbindung II und 400 mg/kg Verbindung I und 360–720 mg/kg Thioverbindung II.

Indikationen für die die erfindungsgemäße Kombination in Betracht kommt:

alle Formen von Krebserkrankungen und Krebsmetastasen insbesondere in großen Körperhöhlen, das heißt mit intrapleuraler und intraperitonealer sowie intravesikaler Lokalisation, beispielsweise Ovarial-Carcinom, sowie auch die adjuvante Chemotherapie des Primärtumors. Pleurametastasen nach Ovarial-Carcinom und Mamma-Carcinom, insbesondere auch intrapleurale und intraperitoneale Krebsmetastasen in Ascites-Form. Disseminierendes Blasen-Carcinom inklusive präcanceröse Stadien und als adjuvante Chemotherapie nach operativer Entfernung des Primärtumors. Knochenweichteiltumoren an Extremitäten insbesondere an Melanomen im Rahmen der regionalen Extremitätenperfusion.

Darüberhinaus sind die erfindungsgemäße Kombination auch für den Einsatz als Immunsuppressiva geeignet.

Insbesondere kommt die erfindungsgemäße Kombination für die loko-regionale Chemotherapie der oben erwähnten Tumore in Frage. Hierzu gehören insbesondere die intracavitäre Chemotherapie und die systemische (zum Beispiel perorale oder parenterale) Anwendung. Unter die intracavitäre Chemotherapie fallen zum Beispiel:

a) die intraperitoneale Anwendung beim Ovarial-Carcinom vom Stadium I–II sowie die Primärtherapie beim Ovarial-Carcinom im Stadium III–IV. Insbesondere gehört hierzu die adjuvante Chemotherapie des Ovarial-Carcinoms nach chirurgischer Entfernung des Primärtumors, die Chemotherapie von Pleurametastasen, vor allem nach Ovarial-Carcinom, Mamma-Carcinom und ähnlichen;

b) die intrapleurale Anwendung bei Pleurametastasen und Ascitiden;

c) die intravesikale Anwendung bei disseminierendem Blasen-Carcinom, inklusive präcanceröse Stadien beziehungsweise als adjuvante Chemotherapie postoperativ;

d) die intrathekale Anwendung bei bestimmten Tumoren des Zentralnervensystems, die wegen der fehlenden Permeabilität der Wirkformen der bekannten Oxazaphosphorin-Cytostatika der Chemotherapie durch letztere nicht zugänglich sind;

e) Anwendung durch regionale Perfusion zum Beispiel bei Knochen- und Weichteiltumoren an Extremitäten, insbesondere bei Melanomen;

f) Autologe Knochenmarkstransplantationen bei der Hochdosis-Chemotherapie von Krebserkrankungen zur Abtötung möglicher Tumormetastasen im Knochenmarksexplantat in vitro und vor der Reimplantation.

Bei systemischer Anwendung (der Wirkstoff wird über die Blutbahn in den Körper gebracht) werden durch die Verwendung der erfindungsgemäßen Kombination kurative Wirkungen erzielt, die vergleichbar sind mit der Wirkung des bekannten Standardmittels Cyclophosphamid. Der Vorteil der erfindungsgemäßen Kombination gegenüber dem Cyclophosphamid und analogen Mitteln liegt darin, daß die 4-Sulfido-oxazaphosphorine bereits direkt cancerotoxisch wirken und daher gezielter und besser dosiert werden können als zum Beispiel das Cyclophosphamid, welches seine Wirkung erst nach einer im Organismus erfolgten enzymatischen Metabolisierung (die über einen längeren Zeitraum erfolgt) entfaltet. Die 4-Sulfido-oxazaphosphorine in Kombination mit den Thioverbindungen der Formel II erlauben daher eine Steuerung in der Pharmakokinetik bei systemischer (zum Beispiel intravenöser) Anwendung und damit eine optimierte systemische Chemotherapie und Polychemotherapie bei Krebserkrankungen.

Schließlich ist durch die erfindungsgemäße Kombination ein praktisch völliges Verschwinden der bekannten urotoxischen Nebenwirkung der Oxazaphosphorine erzielbar, die noch vor der Knochenmarkstoxizität der begrenzende Faktor der Chemotherapie mit Oxazaphosphorinen ist.

So steigt beispielsweise die $DL_{50}$* von 4-(2-Hydroxyethylmercapto)-cyclophosphamid bei lang-

* Mäusestamm, der vom National Medical Research Institut gezüchtet wurde und im Versuchstierhandel erhältlich ist.

* Die $DL_{50}$ ist diejenige Dosis, die bei 50% der eingesetzten Tiere zum Tode führt.

samer intravenöser oder intraperitonealer Gabe bei der Maus auf das 3–4fache (im Falle der Applikation des Cyclohexylaminsalzes auf das 2–3fache), wenn die Injektion in Gegenwart eines 5fachen molaren Überschusses von Cystein erfolgt. Dabei wird der cytotoxische Effekt gegen L1210 Krebszellen** in vitro oder L1210 und S180 Krebszellen in vivo bei intraperitonealer Anwendung nur wenig vermindert. Damit resultiert durch die erfindungsgemäße Kombination eine Verdopplung bis Verdreifachung der therapeutischen Breite im Vergleich zur alleinigen Anwendung des 4-(2-Hydroxy-ethylmercapto)-oxazaphosphorins oder des Cyclohexylaminsalzes ohne Protektor-Thioverbindung der Formel II.

Die 4-Sulfido-oxazaphosphorine der Formel I können bei der Kombination mit den Thioverbindungen II im allgemeinen in einer Dosis von 0,5 mg bis 3500 mg, vorzugsweise 50–2000 mg, insbesondere 200–1400 mg verwendet werden, wobei diese Menge in gelöster Form (beispielsweise in wäßriger Lösung beziehungsweise physiologischer Kochsalzlösung) oder in fester Form (zum Beispiel in einer Tablette oder Kapsel) vorliegen kann. Diese Dosis kann 1 bis 10 mal täglich verabreicht werden.

Die zur Anwendung kommende Menge Thioverbindung der Formel II liegt bezogen auf 1 Mol 4-Sulfido-oxazaphosphorin der Formel I (beziehungsweise dessen Salz) im allgemeinen zwischen 0,2–10 Mol, insbesondere 0,2–5 Mol, vorzugsweise 0,4–5 Mol. Falls die Thioverbindung der Formel II eine $C_2$–$C_6$-Sulfoalkylgruppe enthält, werden beispielsweise 0,2 Mol–3 Mol Verbindung II pro 1 Mol 4-Sulfido-oxazaphosphorin I verwendet. Enthält die Thioverbindung der Formel II keine Sulfoalkylgruppe, werden in der Regel höhere Dosen an Thioverbindung II verwendet, beispielsweise 1–10 Mol Thioverbindung II auf 1 Mol 4-Sulfido-oxazaphosphorin I. Das Mischungsverhältnis von Verbindung I (beziehungsweise deren Salz) zur Thioverbindung II beträgt also 1 Mol:0,2 Mol bis 1 Mol Verbindung I (beziehungsweise deren Salz):10 Mol Thioverbindung II beispielsweise 1:1 bis 1:10 beziehungsweise 1:1 bis 1:5 (jeweils in Mol). Das heißt, werden beispielsweise 337 mg der Verbindung I verwendet (im Falle des 4-(2-Hydroxy-ethylmercapto)-cyclophosphamids = 1 mMol), dann können gleichzeitig oder zu verschiedenen Zeiten beispielsweise 121 mg bis 1210 mg Cystein (1 mMol bis 10 mMol) als Thioverbindung II verwendet werden. Falls in diesem Falle eine andere Thioverbindung der Formel II verwendet wird, errechnen sich entsprechend dem anderen Molgewicht entsprechend andere Dosierungen in mg. Im allgemeinen werden die Thioverbindungen der Formel II in einer Menge von 0,1 mg bis 25 g, vorzugsweise 1 mg bis 5 g, insbesondere 10 mg bis 5 g beziehungsweise 10 mg bis 3,5 g verwendet je in Abhängigkeit von der verwendeten Menge des 4-Sulfido-oxazaphosphorins der Formel I und der jeweils verwendeten speziellen Thioverbindung der Formel II.

Beispielsweise enthalten die pharmazeutischen Zubereitungen zwischen 30–40 mg einer Verbindung der Formel I + 20–100 mg Cystein oder entsprechende Molmengen einer anderen Thioverbindung II und 1230–1300 mg einer Verbindung der Formel I + 1600–4500 mg Cystein (oder entsprechende Molmengen einer anderen Thioverbindung der Formel II). Bevorzugte Anwendungsformen sind Lösungen, die zwischen 0,1 und 13% an aktiver Substanz enthalten, wobei sich diese Angabe sowohl auf ein Gemisch aus einem 4-Sulfido-oxazaphosphorin der Formel I und einer Thioverbindung der Formel II bezieht als auch auf entsprechende Lösungen die jeweils nur einen Bestandteil, das heißt nur das 4-Sulfido-oxazaphosphorin der Formel I und nur die Thioverbindung der Formel II enthalten.

Die Einzeldosis von 4-Sulfido-oxazaphosphorin der Formel I und Thioverbindung der Formel II liegt beispielsweise bei parenteraler Applikation zwischen 0,5 mg Verbindung I + 0,3 mg Verbindung II und 3,5 g Verbindung I und 13 g Thioverbindung II, vorzugsweise zwischen 50 mg Verbindung I + 936 mg Thioverbindung II und 2 g Verbindung I + 7 g Thioverbindung II, insbesondere zwischen 100 mg Verbindung I + 72 mg Thioverbindung II und 1,4 g Verbindung I + 5 g Thioverbindung II. Bei peroraler Applikation liegt die Einzeldosis beispielsweise bei 250 mg Verbindung I + 450 mg Thioverbindung II. Beispielsweise kann bei intravenöser Injektion 1 mal täglich 1 Ampulle von 2–20 ml Inhalt mit 100 mg $P_1$ + 180 mg Cystein (beziehungsweise 162 mg Cyclohexylaminsalz + 146 mg Cystein) bis 1,0 g $P_1$ + 1,8 g Cystein (beziehungsweise 1,62 g Cyclohexylaminsalz + 1,46 g Cystein) empfohlen werden.

Entsprechendes gilt hinsichtlich der anderen 4-Sulfido-oxazaphosphorine der Formel I und der Thioverbindungen der Formel II.

Die akute Toxizität an der NMRI-Maus ($DL_{50}$) der 4-(2-Hydroxy-ethylmercapto-cyclophosphamid/Cystein-Kombination an der Maus liegt beispielsweise bei

intravenöser Applikation bei 600 mg/kg 4-(2-Hydroxy-ethylmercapto-cyclophosphamid + 1100 mg Cystein.

Im allgemeinen liegt die $DL_{50}$ pro kg Maus für die Kombination 4-Sulfido-oxazaphosphorin I + Thioverbindung II zwischen 300 mg bis 1200 mg Verbindung I + 108 mg bis 2,1 g Thioverbindung II, vorzugsweise zwischen 400 mg bis 1000 mg Verbindung I + 290 mg bis 1,8 g Thioverbindung II, insbesondere zwischen 500 mg bis 800 mg Verbindung I + 360 mg bis 1,5 g Thioverbindung II. Die maximale tolerierte Dosis am Menschen für die Kombination 4-Sulfido-oxazaphosphorin I mit Thioverbindung II im Bereich der molaren Mischungsverhältnisse 1:2–1:5 liegt etwa bei 50 mg/kg (Körpergewicht/Mensch), bezogen auf die 4-Sulfido-oxazaphosphorin-Komponente.

** Lymphatische Leukämiezellen der Maus, die als Standard-Krebszellen für Versuchszwecke benutzt werden und bei Tumorbanken (zum Beispiel Deutsches Krebsforschungszentrum in Heidelberg) oder auch im Handel erhältlich sind.

Die Kombination kann in der Humanmedizin und in der Veterinärmedizin allein oder mit anderen pharmakologisch aktiven Stoffen verwendet werden.

Die Verminderung der Toxizität von Verbindungen der Formel I durch die erfindungsgemäße Kombination mit Thioverbindungen der Formel II ist beispielsweise aus folgenden Versuchen ersichtlich:

Akute Toxizität

(Bestimmung nach: Ther, Grundlagen der experimentellen Arzneimittelforschung, Verlagsgesellschaft MBH, Stuttgart, 1965)

Die akute Toxizität von 4-(2-Hydroxy-ethylmercapto)-cyclophosphamid ($P_1$) wurde beispielsweise für intravenöse und intraperitoneale Applikation bei weiblichen NMRI-Mäusen (20–25 g) wie folgt bestimmt:

| Substanz | in mg/kg $DL_{50}$ (30 Tage Beobachtungszeit) | in mg/kg $DL_{50}$ (90 Tage Beobachtungszeit) |
|---|---|---|
| $P_1$ intraperitoneal | 238 | 160 |
| $P_1$ intravenös | 290 | 215 |
| $P_1$ +5fache molare Menge L(+)-Cystein intraperitoneal | 715 | 715 |
| $P_1$ +5fache molare Menge L(+)-Cystein intravenös | 600 | |

Das Cystein wurde dabei in physiologischer NaCl (isotonische Lösung) gelöst und injiziert. Injektionsvolumen stets 20 ml/kg (Cysteinlösungen müssen besonders für intravenöse und intraperitoneale Injektionen frisch bereitet werden).

$P_1$ wird ebenfalls stets in physiologischer NaCl frisch gelöst (0,5–2,5%ige Lösung) und unmittelbar nach der Cystein-Injektion injiziert.

Die angegebenen $DL_{50}$-Werte für die intraperitoneale Injektion von $P_1$ und $P_1$ + Cystein 1 Mol $P_1$:5 Mol Cystein) beziehen sich auf Injektionsvolumina von jeweils 20 ml/kg Körpergewicht. Bei Vergrößerungen des Injektionsvolumens auf das 10fache (200 ml/kg) erhöht sich beispielsweise die $DL_{50}$ für $P_1$ auf 460 mg/kg und die von $P_1$ + Cystein (Molverhältnis 1:5) auf 1090 mg/kg (bezogen auf die $P_1$-Komponente). Bei Erhöhung des Injektionsvolumens bei intraperitonealer Injektion um einen Faktor 10 würde demgemäß eine Verringerung der Gesamttoxizität auf 54–68% der Toxizität bewirkt, die bei dem kleineren Injektionsvolumen bestimmt wurde.

Wie zuvor angegeben, bewirkt die Thioverbindung II bei intraperitonealer Injektion von $P_1$ einen Anstieg der $DL_{50}$ um einen Faktor 3 (30 Tage Beobachtungszeit) beziehungsweise um den Faktor 4,5 (bei 90 Tagen Beobachtungszeit). Bei intravenöser Injektion ist die Toxizitätsverminderung von der Injektionsdauer abhängig. So beträgt bei einer Injektionsdauer von 1 Minute die $DL_{50}$ von $P_1$ + 5fach molare Menge Cystein 600 mg/kg, und sinkt bei schneller Injektion (Bolusinjektion) auf 330 mg/kg, während die $DL_{50}$ von $P_1$ selbst bei 290 mg/kg liegt. Die detoxifizierende Wirkung des Cysteins kommt also bei intravenöser Injektion nur bei längerer (langsamerer) Injektionsdauer (länger als 1 Minute) zum Tragen.

Nach der intraperitonealen Injektion von $P_1$ und Cystein wurden bei den Mäusen weder Schädigungen oder pathologische Veränderungen der Bauchhöhle noch Formveränderungen und Glycogenverlust der Leberzellen oder Gefäßzerstörungen (beispielsweise der subperitonealen Arterien) beobachtet.

Die Verminderung der Toxizität von Verbindungen der Formel I vermindert sich mit steigendem Anteil der Thioverbindungen der Formel II. Der relative Schutzeffekt ist am stärksten im unteren Bereich des Mischungsverhältnisses, beispielsweise in dem Bereich von 1 Mol Gewichtsteil Verbindung I bis zu 2 Mol Gewichtsteilen Thioverbindung der Formel II. In dem anschließenden Bereich (1 Mol Gewichtsteil Verbindung I bis 2–10 Mol Gewichtsteile Thioverbindung II) nimmt die Toxizitätsverminderung relativ langsamer weiter ab.

Subchronische Toxizität

4-(2-Hydroxy-ethylmercapto)-cyclophosphamid ($P_1$) + Cystein (Molverhältnis 1:5) NMRI-Maus.

Bei intraperitonealer Injektion von jeweils 383 mg/kg $P_1$ + 685 mg/kg Cystein (20 ml wäßrige Lösung/kg) entsprechend 53% der $DL_{50}$ für diese Kombination im wöchentlichen Abstand, starben nach 6maliger Injektion 2 von 8 Tieren. Dabei wurden insgesamt 6,8 mMol/kg $P_1$ + 34 mMol/kg Cystein injiziert. Cyclophosphamid nach demselben Schema (ohne Cystein) gegeben, ergab beispielsweise bereits bei 3 mMol/kg Gesamtdosis den Tod von 6 Mäusen.

$P_1$ + Cystein (Molverhältnis 1:5); Hund:

30 mg/kg $P_1$ + 54 mg Cystein (1:5 Mol) wöchentliche intravenöse Injektion (1 ml wäßrige Lösung/kg Hund).

Es wurde keine Aktivitätsbeeinträchtigung und nur geringer Gewichtsverlust beobachtet. Töten des Tieres nach 4 Wochen mit der Gesamtdosis von 120 mg/kg $P_1$ + 216 mg Cystein. Sektion: Kein makroskopischer Befund, insbesondere kein Hinweis für eine Cystitis. Im Blut geringer Abfall von Leukocyten und Thrombocyten.

Lokale Toxizität

Kaninchen-Harnblase ($P_1$ + Cystein 1:5 Mol):

Einmalige Instillation (Einführen von Flüssigkeit in ein Organ über eine gewisse Zeitspanne mittels Katheter) von 1,35 g $P_1$ + 2,42 g Cystein in 25 ml aqua destillata (Löslichkeitsgrenze) in die Harnblase von weiblichen Kaninchen-Bastarden (ca. 4 kg Gewicht) für 30 Minuten; Verweildauer: 30 Minuten, Histologischer Befund 3 Tage nach Instillation: Unauffällige Blasenschleimhaut ohne erkennbare Anzeichen von Cystitis. Tier lebte noch 10 Monate nach der Instillation.

Dreimalige Instillation in jeweils 1 Tag Abstand in die Harnblase von weiblichen Kaninchen mit implantierten Brown-Pearce-Sarcomen: Sonstige Bedingungen wie oben.

Lokaltoxizität makroskopisch: Geringe Verdikkung der Blasenwand, keine Einblutungen, keine beziehungsweise nur geringfügige Nekrotisierung.

Histologisch: Urothel in weiten Bezirken intakt, stellenweise geringfügige ödematöse und entzündliche Schädigungen außerhalb des Tumorimplantats im Bereich des normalen Urothels erkennbar.

Dreimalige Instillation in täglicher Aufeinanderfolge, sonstige Bedingungen wie oben.

Makroskopischer Befund: Verdickte Blasenwand mit Einblutungen und wenigen nekrotischen Bezirken, histologisch: nur wenig Urothel vorhanden, Blasenwand ödematös entzündlich verändert.

Hundeblase: weiblichem Beagle (Hund) ca. 15 kg Gewicht, wurden jeweils 50 ml einer Lösung von 2,7 g $P_1$ + 4,84 g Cystein in 50 ml aqua destillata bei Raumtemperatur injiziert. Verweildauer: 30 Minuten. Cytoskopischer Befund: jeweils 1 Woche nach Instillation: Blasenschleimhaut unauffällig, keine Anzeichen für Cystitis. Wiederholung der Instillation nach 3 Monaten.

Cytoskopie nach 1 Woche nach der Instillation: Unauffällige Blasenschleimhaut, keine Anzeichen für Cystitis.

Die pharmakologische Wirkung der erfindungsgemäßen Kombination und der 4-Sulfido-oxazaphosphorine der Formel I ist beispielsweise aus folgenden Versuchen ersichtlich:

Lokale (intracavitäre) Chemotherapie

a) Bei Leukämie (L1210-Leukämie-Zellen) an der Maus:

0,5 Stunden nach Verimpfung von $5 \times 10^5$ L1210-Leukämiezellen pro Maus ($DBA_2$/Han♀*) wurde durch einmalige intraperitoneale Injektion von $P_1$ (curative Dosis 68 mg/kg) beziehungsweise $P_1$ + Cystein (Molverhältnis 1:5) therapiert. Die curative Dosis für $P_1$ bei der Kombination mit dem Cystein war 90 mg/kg. Die therapeutischen Breiten $TI_{50}$ (Quotient aus $DL_{50}$ und $DC_{50}$) betragen für $P_1$ allein: 3,5 und für $P_1$ + Cystein hingegen 7,3. Es wurden jeweils physiologische Kochsalzlösungen verwendet (20 ml Lösung/kg Maus). Die Injektionsdauer war stets kürzer als 1 Minute.

b) Beim S180 Sarkom in der Ascites-Form (NMRI-Maus):

Therapie durch einmalige intraperitoneale Injektion (Injektionsdauer kürzer als 1 Minute) von $P_1$ beziehungsweise $P_1$ + Cystein in physiologischer Kochsalzlösung (Molverhältnis 1:5; 10 ml Lösung pro kg Maus) 3 Tage nach Transplantation von $10^6$ Tumorzellen pro Maus. Für $P_1$ allein betrug die $DC_{50}$ 18 mg/kg; für $P_1$ + Cystein (Molverhältnis 1:5) 32 mg/kg; die entsprechenden $TI_{50}$-Werte für $P_1$ sind 13 für 30 Tage und 9,7 für 60 Tage Beobachtungszeit; für die Kombination $P_1$ + Cystein (Molverhältnis 1:5) ergibt sich hingegen ein Wert von 23 für 60 Tage Beobachtungszeit. In beiden Tumoren erreicht man also ungefähr eine Verdoppelung der therapeutischen Breite durch Cysteinzugabe.

c) Brown-Pearce-Sarkom in der Harnblase von Kaninchen:

Kaninchen-Bastarden wurden ca. $10^7$ Zellen eines Brown-Pearce-Sarkoms unter die Submukosa (Zellschicht unter einer Schleimhaut) implantiert. 4 Tage nach Tumorimplantation Beginn der intracavitären Chemotherapie durch Instillation von 160 Millimol $P_1$ + 800 Millimol Cystein (Molverhältnis 1:5) in 25 ml aqua destillata von 37 °C für 30–60 Minuten. Insgesamt 3malige Wiederholung mit jeweils 1 Tag Intervall.

Histologische Schnitte der Blasenwand zeigen ein deutliches Ansprechen des Tumors auf die Therapie mit großflächigen nekrotischen Bezirken. Charakteristisch ist die Nekrotisierung von der Peripherie des Tumorimplantats her im Gegensatz zu spontanen, zentral gelegenen Nekrosen unbehandelter Kontrollen. Die Tumorgröße betrug 5–25 mm Durchmesser. Bei Beendigung des Versuchs ist das Endothel tumorfrei ohne histologische Veränderungen.

Systemische Chemotherapie (der Wirkstoff wird über die Blutbahn in den Körper gebracht) mit menschlichen Tumorzellen (Tumorxenograften, Übertragung menschlicher Tumorzellen auf Versuchstiere) auf tymusaplastischen nu/nu-Mäusen*

(Die experimentelle Durchführung erfolgte gemäß: Povlsen, C.O., Jacobsen, G.K., Rygaard, J., The Laboratory Animal in Drug Testing, Editor A. Spiegel, Seiten 63–73, Fischer-Verlag, Stuttgart, 1973).

a) Menschliches Mamma-Carcinom aus Operationsmaterial einer Frauenklinik wurde auf thymusaplastische nu/nu-Mäusen heterotransplantiert. Nach der 23.–25. Passage jeweils durch Implantation einer dünnen Tumorscheibe von 8–10 mm Durchmesser in die Milchleiste der Tiere wurde nach Anwachsen der Implantate auf 0,15–0,25 g therapiert und der kurative Effekt durch Ausmessen der Tumorfläche bestimmt.

---

* bestimmter Mäusestamm von weiblichen Mäusen, die für Versuchszwecke in der Tumorforschung verwendet werden und beispielsweise vom Zentralinstitut für Versuchstierforschung in Hannover bezogen werden können.

* nu/nu-Mäuse = Nacktmäuse, die den genetischen Mangel der fehlenden Immunabwehr haben

Nach einmaliger intraperitonealer Injektion (Injektionsdauer kürzer als 1 Minute) von 10 ml einer wäßrigen Lösung von 98 mg $P_1$ + 176 mg Cystein/kg Maus deutliche Verzögerung des Tumorwachstums im Vergleich zu den unbehandelten Kontrollen. Bei einmaliger intraperitonealer Injektion (Dauer kürzer als 1 Minute) von 296 mg/kg $P_1$ + 535 mg Cystein (entsprechend ca. 40% der $DL_{50}$) in wäßriger Lösung (10 ml/kg Maus) erfolgt eine Hemmung des Tumorwachstums, die derjenigen entspricht, die mit einmaliger Injektion von 100 mg/kg Cyclophosphamid in physiologischer Kochsalzlösung (10 ml/kg Maus) als Standard-Vergleichssubstanz erzielt werden kann, während die unbehandelten Kontrollen während der gleichen Beobachtungszeit (3 Wochen) auf die 4fache Tumoroberfläche mit der entsprechenden proportionalen Gewichtszunahme kommen.

Bei Mehrfachinjektionen von jeweils 250 ml/kg $P_1$ + 450 mg/kg Cystein (wäßrige Lösung jeweils 10 ml/kg Maus) jeweils in wöchentlichem Abstand intraperitoneal injiziert (Injektionsdauer kürzer als 1 Minute), wird in einem Zeitraum von 5 Wochen eine Verringerung der Tumormasse im Sinne einer echten kurativen Heilung erreicht, die der Wirkung von 150 mg/kg Cyclophosphamid, ebenfalls in wöchentlichem Abstand injiziert, entspricht. Die unbehandelten Kontrollen sterben innerhalb des Beobachtungszeitraums von 5 Wochen am Tumor.

Die (subchronischen) toxischen Wirkungen von $P_1$ in Kombination mit Cystein sind jedoch bei dieser Anwendung deutlich geringer als die von Cyclophosphamid, was durch den erheblich besseren Allgemeinzustand der behandelten Tiere erkennbar ist.

b) Menschliches verhornendes Blasencarzinom auf der nu/nu-Maus:

Ein Chemotherapie-resistentes weibliches verhornendes Blasencarzinom aus dem Operationsmaterial einer urologischen Klinik wurde nach der 4. Passage auf männliche nu/nu-Mäuse transplantiert. Systemische Chemotherapie erfolgte durch 5malige intraperitoneale Injektion von 250 mg/kg $P_1$ (= 0,74 mM/kg Maus) + 449 mg/kg Cystein (wäßrige Lösung; 10 ml/kg Maus) in jeweils wöchentlichem Abstand beziehungsweise 5malige intraperitoneale Injektion von Cyclophosphamid (150 mg/kg = 0,53 mMol/kg) in gleichem Abstand. Im Vergleich zu den Kontrollen, die 18 Tage nach Versuchsbeginn gestorben sind, bewirkt $P_1$ + 5fach molare Menge Cystein bei wiederholter Anwendung einen Stillstand des Turmorwachstums und Überleben der Tiere. Der cancerostatische Effekt entspricht demjenigen von Cyclophosphamid als Standardvergleichssubstanz in einer Dosierung von 150 mg/kg entsprechend 2/3 Moläquivalenten.

Die isolierte Extremitätenperfusion ist ein Verfahren zur cytostatischen Behandlung maligner Tumoren der Extremitäten, wobei der übrige Organismus der Wirkung von Cytostatika nicht ausgesetzt ist.

Als Tiermodell für dieses Verfahren dient die isolierte Perfusion des tumortragenden (Yoshida Sarkom) Rattenbeines, wobei das Tumorwachstum nach Leerperfusion und Cytostatikaperfusion gemessen wird.

Nach 20minütiger Perfusion mit $P_1$ (5000 nmol/ml) + Cystein (5fach molar) wird eine vollständige Heilung der Tiere erzielt, ebenso bei Perfusion mit einer Lösung von 10 000 nmol/ml. Die Tiere waren ein halbes Jahr nach dem Versuch noch rezidivfrei.

Perfusion mit $P_1$ allein war weniger erfolgreich. Bei 5000 nmol/ml starben die Tiere nach der Perfusion. Bei 2000 nmol/ml war neben einer Verzögerung des Tumorwachstums eine Heilung lediglich bei zwei von 4 Tieren zu beobachten.

Die Herstellung der pharmazeutischen Zubereitungen, die die 4-Sulfido-oxazaphosphorine der Formel I und/oder Thioverbindungen der Formel II enthalten, geschieht nach allgemein bekannten Methoden, wie nachfolgend beispielsweise für verschiedene Injektionslösungen erläutert wird:

Beispiel 1

Kombinationspräparate, enthaltend als Wirkstoffe jeweils 50 mg (= 0,15 Millimol) 4-(-2-Hydroxy-ethylmercapto)-cyclophosphamid ($P_1$) und L-Cystein in unterschiedlichen molaren Verhältnissen (2 ×, 5 × und 10 ×) bezogen auf die Substanz $P_1$ gemäß folgender Übersicht:

Eine Injektionsflasche enthält

| | Zubereitung 1 | Zubereitung 2 | Zubereitung 3 |
|---|---|---|---|
| Substanz $P_1$ | 50,0 mg (0,15 Millimol) | 50,0 mg | 50,0 mg |
| L-Cystein | 36,0 mg (0,3 Millimol) | 90,0 mg (0,75 Millimol) | 180,0 mg (1,5 Millimol) |
| D-Mannit | 200,0 mg | 110,0 mg | – |
| Wasser für Injektionszwecke ad | 2,0 ml | 2,0 ml | 2,0 ml |

Die Substanzen $P_1$, L-Cystein und – falls erforderlich – D-Mannit werden jeweils unter Begasung mit Stickstoff in soviel Wasser gelöst, daß eine Lösung von 2 ml Volumen entsteht und in bekannter Weise einer Sterilfiltration unterzogen. Diese Lösung wird dann unter aseptischen Bedingungen in braune 10 ml Injektionsflaschen dosiert, mit Gefriertrocknungsstopfen versehen und in einer Gefriertrocknungsanlage lyophilisiert. Anschließend wird die Gefriertrocknungsanlage mit trockenem Stickstoff belüftet und die Ampullenflaschen in der Anlage verschlossen.

Zur Herstellung der applizierbaren Injektionslösung wird der Inhalt der Ampullenflaschen in 5 ml Wasser für Injektionszwecke aufgelöst.

Zubereitungen für die getrennte Anwendung von 4-Sulfio-oxazaphosphorinen I und Thioverbindungen II.

Beispiel 2

Wie unter Beispiel 1 angegeben, werden 50,0 mg (0,15 Millimol) 4-(2-Hydroxy-ethylmercapto)-cyclophosphamid ($P_1$) und 250,0 mg D-Mannit mit Wasser (für Injektionszwecke) zu einer Lösung von 2 ml Volumen gelöst und lyophilisiert.

Beispiel 3

Wie unter Beispiel 1 angegeben, werden 36,0 mg (0,3 Millimol) Cystein und 220,0 mg D-Mannit mit Wasser (für Injektionszwecke) zu einer Lösung von 2 ml Volumen gelöst und lyophilisiert.

Auf dieselbe Weise werden Lyophilsate des Cysteins hergestellt, die

90 mg (0,75 Millimol) L-Cystein und
140,0 mg D-Mannit sowie
180 mg (1,5 Millimol) L-Cystein (ohne Mannitzusatz) enthalten.

Zur Herstellung von Injektionslösungen wird der Inhalt der Ampullenflaschen gemäß den Beispielen 2 und 3 in jeweils 5 ml Wasser (für Injektionszwecke) aufgelöst.

Beispiel 4

Kombinationspräparate, enthaltend als Wirkstoffe jeweils 82 mg (= 0,15 Millimol) «Cyclohexylaminsalz» und L-Cystein in unterschiedlichen molaren Verhältnissen (2 ×, 5 × und 10 ×) bezogen auf die Substanz «Cyclohexylaminsalz» gemäß folgender Übersicht:

Cyclohexylaminsalz = 3-[2-(Bis-(2-chlorethyl)-amino-2-oxo-tetrahydro-2H-1,3,2-oxazaphosphorin-4-yl-thio]-2-mercaptopropansulfonsäure-cyclohexylaminsalz.

Eine Injektionsflasche enthält

| | Zubereitung 1 | Zubereitung 2 | Zubereitung 3 |
|---|---|---|---|
| Substanz «Cyclohexylaminsalz» | 82 mg (0,15 Millimol) | 82 mg | 82 mg |
| L-Cystein | 36,0 mg (0,3 Millimol) | 90,0 mg (0,75 Millimol) | 180,0 mg (1,5 Millimol) |
| D-Mannit | 200,0 mg | 110,0 mg | – |
| Wasser für Injektionszwecke ad | 2,0 ml | 2,0 ml | 2,0 ml |

Die Substanzen «Cyclohexylaminsalz», L-Cystein und – falls erforderlich – D-Mannit werden jeweils unter Begasung mit Stickstoff in soviel Wasser gelöst, daß eine Lösung von 2 ml Volumen entsteht und in bekannter Weise einer Sterilfiltration unterzogen. Diese Lösung wird dann unter aseptischen Bedingungen in braune 10 ml Injektionsflaschen dosiert, mit Gefriertrocknungsstopfen versehen und in einer Gefriertrocknungsanlage lyophilisiert. Anschließend wird die Gefriertrocknungsanlage mit trockenem Stickstoff belüftet und die Ampullenflaschen in der Anlage verschlossen.

Zur Herstellung der applizierbaren Injektionslösung wird der Inhalt der Ampullenflaschen in 5 ml Wasser für Injektionszwecke aufgelöst.

Beispiel 5

(Zubereitungen für die getrennte Anwendung von 4-Sulfio-oxazaphosphorinen I und Thioverbindungen II)

Wie unter Beispiel 4 angegeben, werden 82 mg (0,15 Millimol) «Cyclohexylaminsalz» und 250,0 mg D-Mannit mit Wasser (für Injektionszwecke) zu einer Lösung von 2 ml Volumen gelöst und lyophilisiert.

Wie unter Beispiel 4 angegeben, werden 36,0 mg (0,3 Millimol) Cystein und 220,0 mg D-Mannit mit Wasser (für Injektionszwecke) zu einer Lösung von 2 ml Volumen gelöst und lyophilisiert.

Auf dieselbe Weise werden Lyophilsate des Cysteins hergestellt, die

90 mg (0,75 Millimol) L-Cystein und
140,0 mg D-Mannit sowie
180 mg (1,5 Millimol) L-Cystein (ohne Mannitzusatz) enthalten.

Zur Herstellung von Injektionslösungen wird der Inhalt der Ampullenflaschen mit den Lyophilisaten in jeweils 5 ml Wasser (für Injektionszwecke) aufgelöst.

Herstellungsbeispiel 1

2-(2-Chlor-ethylamino)-3-(2-chlor-ethyl)-4-(2-hydroxyethylthio)-tetrahydro-2H-1,3,2-oxazaphosphorin-2-oxid

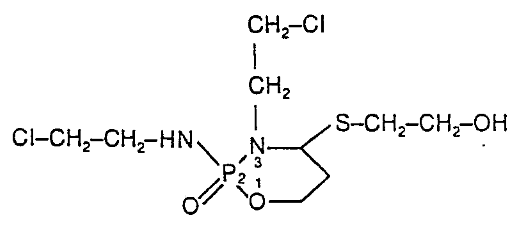

5,4 g (19 mMol) 4-Hydroxyphosphamid werden in 40 ml Methylenchlorid suspendiert, mit 2,8 ml (39 mMol) 2-Mercaptoethanol versetzt, auf 5 °C

abgekühlt, unter Rühren 150 ml Trichloressigsäure zugesetzt, nach 3 Tagen bei 4 °C eingeengt, das Öl an Silicagel mit den Eluentien Chloroform/Methanol (15:1) und Aceton/Methylenchlorid (3:1) gereinigt.

Ausbeute: 3,4 g (53% der Theorie)
$R_f$-Wert 0,45
Eluens: Chloroform/Methanol (5:1)
Anfärbung: Jod.

Herstellungsbeispiel 2

2-(Bis-(2-chlor-ethylamino)-4-[3-hydroxy-2-mercaptopropyl-(1)-thio]-tetrahydro-2H-1,3,2-oxazaphosphorin-2-oxid

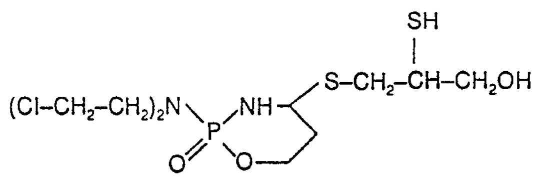

4-Hydroxy-cyclophosphamid wird mit der 5fachen Menge 2,3-Mercapto-1-propanol in Aceton bei 4 °C umgesetzt. Nach 2 Stunden wird Aceton im Wasserstrahlvakuum unter Kühlung im Eisbad abgezogen. Der Rückstand wird auf einer Kieselgelplatte durch präparative Dünnschichtchromatographie mit Essigester/Aceton (1:1) als Laufmittel getrennt. $R_f$ = 0,4; Nachweis der alkylierenden Aktivität (γ-Nitrobenzylpyridin) und Jod-Acid auf Thio-Gruppen. Die Zone bei $R_f$ 0,4 wird mit Methylenchlorid in der Kälte extrahiert und durch Fällung mit Diethylether das mikrokristalline Rohprodukt erhalten. Schmelzpunkt (unkorrigiert): 90–95 °C.

**Patentansprüche**

1. Verwendung von 4-Sulfido-oxazaphosphorinen der allgemeinen Formel

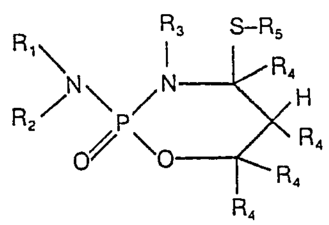

I

worin

$R_1$, $R_2$ und $R_3$, die gleich oder verschieden voneinander sein können, Wasserstoff, Methyl, Ethyl, 2-Chlorethyl oder 2-Methansulfonyloxyethyl darstellen und dabei mindestens zwei dieser Reste 2-Chlorethyl und/oder 2-Methansulfonyloxyethyl sind,

$R_4$ Wasserstoff oder Methyl ist und

$R_5$ ein $C_2$–$C_6$-Hydroxy-alkylrest oder ein $C_2$–$C_6$-Mercaptoalkylrest ist, wobei jeder dieser Reste auch eine zusätzliche Mercaptogruppe enthalten kann oder worin $R_5$ eine Carboxy-$C_1$–$C_{10}$-alkylgruppe, eine Carb-$C_1$–$C_6$-alkoxy-$C_1$–$C_{10}$-alkylgruppe, eine 2-Amino-2-carboxy-ethylgruppe, eine 2-Amino-2-carb-$C_1$–$C_6$-alkoxy-ethylgruppe, eine 2-$C_2$–$C_6$-Alkanoylamino-2-carboxyethylgruppe, eine 2-$C_2$–$C_6$-Alkanoylamino-2-carb-$C_1$–$C_6$-alkoxy-ethylgruppe, den Glutathionylrest, eine $C_2$–$C_6$-Sulfoalkylgruppe oder eine $C_2$–$C_6$-Sulfoalkylgruppe, welche eine Mercaptogruppe enthält, bedeutet sowie deren physiologisch verträglichen Salzen in Kombination mit Thioverbindungen der allgemeinen Formel

$$R_6SR_7 \qquad II$$

worin $R_6$ der Glutathionylrest ist oder einen geraden oder verzweigten $C_2$–$C_6$-Alkylrest darstellt, welcher ein- oder zweifach durch Hydroxygruppen, Mercaptogruppen, Aminogruppen, $C_2$–$C_6$-Alkanoylaminogruppen, Sulfogruppen, Carb-$C_1$–$C_6$-alkoxygruppen oder Carboxygruppen substituiert ist und $R_7$ Wasserstoff bedeutet oder worin $R_7$ auch eine $C_2$–$C_6$-Sulfoalkylthiogruppe sein kann, falls $R_6$ eine $C_2$–$C_6$-Sulfoalkylgruppe darstellt beziehungsweise den physiologisch verträglichen Salzen solcher Thioverbindungen der Formel II zur Herstellung eines Arzneimittels für die therapeutische Anwendung zur Bekämpfung von Krebs und zur Immunsuppression.

2. Arzneimittel, enthaltend mindestens ein 4-Sulfido-oxazaphosphorin der Formel I (definiert wie in Anspruch 1) oder dessen physiologisch verträgliches Salz und mindestens eine Thioverbindung der Formel II (definiert wie in Anspruch 1) oder deren physiologisch verträgliches Salz.

3. Arzneimittel nach Anspruch 2, dadurch gekennzeichnet, daß die Menge an 4-Sulfido-oxazaphosphorin der Formel I zwischen 0,5–3500 mg liegt und die Menge an Thioverbindung der Formel II 0,2–10 Mol (in mg) beträgt, bezogen auf jeweils 1 Mol des verwendeten 4-Sulfido-oxazaphosphorins.

4. Arzneimittel nach Anspruch 2 oder 3, dadurch gekennzeichnet, daß es als Wirkstoff mindestens ein 4-Sulfido-oxazaphosphorin der Formel I oder dessen physiologisch verträgliches Salz und mindestens eine Thioverbindung der Formel II oder deren physiologisch verträgliches Salz zusammen mit einem üblichen pharmazeutischen Träger- und/oder Verdünnungsmittel enthält.

5. Therapeutische Packung, dadurch gekennzeichnet, daß sie in zwei getrennten galenischen Zubereitungsformen

a) mindestens ein 4-Sulfido-oxazaphosphorin der Formel I (definiert wie in Anspruch 1) oder dessen physiologisch verträgliches Salz allein oder zusammen mit einem üblichen pharmazeutischen Träger- und/oder Verdünnungsmittel und

b) mindestens eine Thioverbindung der Formel II (definiert wie in Anspruch 1) oder deren physiologisch verträgliches Salz allein oder zusammen mit einem üblichen pharmazeutischen Träger- und/oder Verdünnungsmittel enthält.

6. Verfahren zur Herstellung eines Arzneimittels oder einer therapeutischen Packung nach einem oder mehreren der vorangegangenen Ansprüche, dadurch gekennzeichnet, daß mindestens ein 4-Sulfido-oxazaphosphorin der Formel I (definiert wie in Anspruch 1) oder deren physiologisch verträgliches Salz und mindestens eine Thioverbindung der Formel II (definiert wie in Anspruch 1) oder deren physiologisch verträgliches Salz zusammen oder jeweils getrennt mit gebräuchlichen pharmazeutischen Trägerstoffen beziehungsweise Verdünnungsmitteln zu pharmazeutischen Zubereitungen verarbeitet wird.

**Claims**

1. The use of 4-sulfido-oxazaphosphorines corresponding to the following general formula

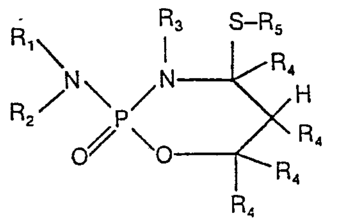

I

in which

$R_1$, $R_2$ and $R_3$ may be the same or different and represent hydrogen, methyl, ethyl, 2-chloroethyl or 2-methanesulfonyloxyethyl and at least two of these substituents are 2-chloroethyl and/or 2-methanesulfonyloxyethyl,

$R_4$ is hydrogen or methyl and

$R_5$ is a $C_2$–$C_6$ hydroxyalkyl radical or a $C_2$–$C_6$ mercaptoalkyl radical, each of these radicals optionally containing an additional mercapto group, or in which $R_5$ is a carboxy-$C_1$–$C_{10}$-alkyl group, a carb-$C_1$–$C_6$-alkoxy-$C_1$–$C_{10}$-alkyl group, a 2-amino-2-carboxyethyl group, a 2-amino-2-carb-$C_1$–$C_6$-alkoxyethyl group, a 2-$C_2$–$C_6$-alkanoylamino-2-carboxyethyl group, a 2-$C_2$–$C_6$-alkanoylamino-2-carb-$C_1$–$C_6$-alkoxyethyl group, the glutathionyl radical, a $C_2$–$C_6$ sulfoalkyl group or a $C_2$–$C_6$ sulfoalkyl group containing a mercapto group, or physiologically acceptable salts thereof in combination with thio compounds corresponding to the following general formula

$$R_6SR_7 \qquad II$$

in which

$R_6$ is the glutathionyl radical or represents a linear or branched $C_2$–$C_6$ alkyl radical mono- or di-substituted by hydroxy groups, mercapto groups, amino groups, $C_2$–$C_6$ alkanoylamino groups, sulfo groups, carb-$C_1$–$C_6$-alkoxy groups or carboxy groups and $R_7$ is hydrogen or in which $R_7$ may also be a $C_2$–$C_6$ sulfoalkylthio group where $R_6$ is a $C_2$–$C_6$ sulfoalkyl group,

or the physiologically acceptable salts of thio compounds of formula II for the production of a medicament for therapeutic use in the treatment of cancer and in immunosuppression.

2. A medicament containing at least one 4-sulfido-oxazaphosphorine of formula I (as defined in claim 1) or a physiologically acceptable salt thereof and at least one thio compound of formula II (as defined in claim 1) or a physiologically acceptable salt thereof.

3. A medicament as claimed in claim 2, characterized in that the quantity of 4-sulfido-oxazaphosphorine of formula I is between 0.5 and 3500 mg while the quantity of thio compound of formula II is 0.2 to 10 mol (in mg), based in either case on 1 mol of the 4-sulfido-oxazaphosphorine used.

4. A medicament as claimed in claim 2 or 3, characterized in that it contains as active principle at least one 4-sulfido-oxazaphosphorine of formula I or a physiologically acceptable salt thereof and at least one thio compound of formula II or a physiologically acceptable salt thereof together with a standard pharmaceutical excipient and/or diluent.

5. A therapeutic pack, characterized in that it contains in two separate galenic formulations

a) at least one 4-sulfido-oxazaphosphorine of formula I (as defined in claim 1) or a physiologically acceptable salt thereof either on its own or together with a standard pharmaceutical excipient and/or diluent and

b) at least one thio compound of formula II (as defined in claim 1) or a physiologically acceptable salt thereof either on its own or together with a standard pharmaceutical excipient and/or diluent.

6. A process for the preparation of a medicament or a therapeutic pack according to one or more of the preceding claims, characterized in that at least one 4-sulfido-oxazaphosphorine of formula I (as defined in claim 1) or a physiologically acceptable salt thereof and at least one thio compound of formula II (as defined in claim 1) or a physiologically acceptable salt thereof are processed together or separately with standard pharmaceutical excipients or diluents to form pharmaceutical preparations.

**Revendications**

1. Utilisation de 4-sulfido-oxazaphosphorines de formule générale

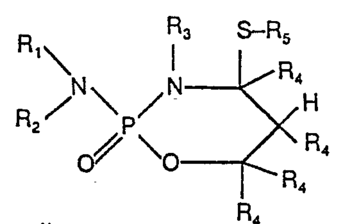

I

dans laquelle

$R_1$, $R_2$ et $R_3$, qui peuvent être identiques ou différents les uns des autres, représentent chacun un atome d'hydrogène, un groupement méthyle, éthyle, 2-chloréthyle ou 2-méthanesulfonyloxyéthyle, deux au moins de ces radicaux étant des groupements 2-chloréthyle et/ou 2-méthanesulfonyloxyéthyle,

$R_4$ est un atome d'hydrogène ou un groupement méthyle et

$R_5$ est un radical $C_2$–$C_6$-hydroxyalkyle ou un radical $C_2$–$C_6$-mercaptoalkyle, chacun de ces radicaux pouvant aussi contenir un groupement mercapto supplémentaire, ou dans laquelle $R_5$ représente un groupement carboxy-$C_1$–$C_{10}$-alkyle, un groupement carb-$C_1$–$C_6$-alcoxy-$C_1$–$C_{10}$-alkyle, un

groupement 2-amino-2-carboxyéthyle, un groupement 2-amino-2-carb-$C_1$–$C_6$-alcoxyéthyle, un groupement 2-$C_2$–$C_6$-alcanoylamino-2-carboxyéthyle, un groupement 2-$C_2$–$C_6$-alcanoylamino-2-carb-$C_1$–$C_6$-alcoxyéthyle, le radical glutathionyle, un groupement $C_2$–$C_6$-sulfoalkyle ou un groupement $C_2$–$C_6$-sulfoalkyle qui contient un groupement mercapto, ainsi que de leurs sels physiologiquement acceptables, en combinaison avec des composés thio de formule générale

$$R_6SR_7 \qquad II$$

dans laquelle $R_6$ est le radical glutathionyle ou représente un radical alkyle en $C_2$–$C_6$ à chaîne droite ou ramifiée qui est substitué une ou deux fois par des groupements hydroxyle, des groupements mercapto, des groupements amino, des groupements $C_2$–$C_6$-alcanoylamino, des groupements sulfo, des groupements carb-$C_1$–$C_6$-alcoxy ou des groupements carboxy, et $R_7$ représente un atome d'hydrogène, ou dans laquelle $R_7$ peut aussi être un groupement $C_2$–$C_6$-sulfoalkylthio dans le cas où $R_6$ représente un groupement $C_2$–$C_6$-sulfoalkyle, ou avec les sels physiologiquement acceptables de tels composés thio de formule II, pour la préparation d'un médicament à usage thérapeutique pour la lutte contre le cancer et pour l'immunosuppression.

2. Médicament, contenant au moins une 4-sulfido-oxazaphosphorine de formule I (telle que définie dans la revendication 1) ou un sel physiologiquement acceptable de celle-ci et au moins un composé thio de formule II (tel que défini dans la revendication 1) ou un sel physiologiquement acceptable de celui-ci.

3. Médicament selon la revendication 2, caractérisé en ce que la quantité de 4-sulfido-oxazaphosphorine de formule I se situe entre 0,5 et 3500 mg et la quantité de composé thio de formule II s'élève à 0,2–10 mol (en mg) sur la base de 1 mol de la 4-sulfido-oxazaphosphorine utilisée.

4. Médicament selon la revendication 2 ou 3, caractérisé en ce qu'il contient, en tant que substance active, au moins une 4-sulfido-oxazaphosphorine de formule I ou un sel physiologiquement acceptable de celle-ci et au moins un composé thio de formule II ou un sel physiologiquement acceptable de celui-ci, en combinaison avec un excipient et/ou un diluant pharmaceutique usuel.

5. Ensemble thérapeutique, caractérisé en ce qu'il contient, sous deux formes de préparation galénique séparées,

a) au moins une 4-sulfido-oxazaphosphorine de formule I (telle que définie dans la revendication 1) ou un sel physiologiquement acceptable de celle-ci, seul ou en combinaison avec un excipient et/ou un diluant pharmaceutique usuel et

b) au moins un composé thio de formule II (tel que défini dans la revendication 1) ou un sel physiologiquement acceptable de celui-ci, seul ou en combinaison avec un excipient et/ou un diluant pharmaceutique usuel.

6. Procédé de préparation d'un médicament ou d'un ensemble thérapeutique selon l'une quelconque des revendications 2 à 5, caractérisé en ce qu'au moins une 4-sulfido-oxazaphosphorine de formule I (telle que definie dans la revendication 1) ou un sel physiologiquement acceptable de celle-ci et au moins un composé thio de formule II (tel que défini dans la revendication 1) ou un sel physiologiquement acceptable de celui-ci sont transformés ensemble ou chacun séparément, en combinaison avec des excipients ou des diluants pharmaceutiques usuels, en préparations pharmaceutiques.